# EUROPEAN PATENT SPECIFICATION

(11) **EP 3 426 768 B1**
(45) Date of publication and mention of the grant of the patent: **16.06.2021**
(21) Application number: 16729045.1
(22) Date of filing: 07.03.2016
(51) Int. Cl.: C12N 5/071, C12N 5/0735, C12N 5/074

(54) **METHOD OF DIFFERENTIATING PLURIPOTENT STEM CELLS**
VERFAHREN ZUR DIFFERENZIERUNG PLURIPOTENTER STAMMZELLEN
METHOD OF DIFFERENTIATING PLURIPOTENT STEM CELLS

(43) Date of publication of application: 16.01.2019
(73) Proprietor: Centre National de la Recherche Scientifique (CNRS), 75016 Paris (FR); The Foundation for the Promotion of Industrial Science, Tokyo 153-8505 (JP)
(72) Inventor: LECLERC, Eric, Tokyo 113-0033 (JP); SAKAI, Yasuyuki, Tokyo 113-0033 (JP); KIMURA, Keiichi, Tokyo 113-0033 (JP); MIYAJIMA, Atsushi, Tokyo 113-0033 (JP); KIDO, Taketomo, Tokyo 113-0033 (JP)
(74) Representative: Regimbeau
(86) International application number: PCT/IB2016/000807
(87) International publication number: WO 2017/153802

(56) References cited:
- LOUISE SIVERTSSON ET AL: "Hepatic Differentiation and Maturation of Human Embryonic Stem Cells Cultured in a Perfused Three-Dimensional Bioreactor", STEM CELLS AND DEVELOPMENT, vol. 22, no. 4, 15 February 2013 (2013-02-15), pages 581-594, XP055273448, NL ISSN: 1547-3287, DOI: 10.1089/scd.2012.0202
- RICHARD L. GIESECK III ET AL: "Maturation of Induced Pluripotent Stem Cell Derived Hepatocytes by 3D-Culture", PLOS ONE, vol. 9, no. 1, 22 January 2014 (2014-01-22), page e86372, XP055307825, DOI: 10.1371/journal.pone.0086372
- RÉGIS BAUDOIN ET AL: "Investigation of expression and activity levels of primary rat hepatocyte detoxication genes under various flow rates and cell densities in microfluidic biochips", BIOTECHNOLOGY PROGRESS., vol. 30, no. 2, 11 January 2014 (2014-01-11), pages 401-410, XP055307691, US ISSN: 8756-7938, DOI: 10.1002/btpr.1857
- BAUDOIN ET AL: "Evaluation of seven drug metabolisms and clearances by cryopreserved human primary hepatocytes cultivated in microfluidic biochips", XENOBIOTICA, TAYLOR AND FRANCIS, LONDON, GB, vol. 43, 20 February 2013 (2013-02-20), pages 140-152, XP009191941, ISSN: 0049-8254 [retrieved on 2012-07-25]
- RÉGIS BAUDOIN ET AL: "Parallelized microfluidic biochips in multi well plate applied to liver tissue engineering", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 173, 1 October 2012 (2012-10-01), pages 919-926, XP055307563, NL ISSN: 0925-4005, DOI: 10.1016/j.snb.2012.06.050

## Description

### [Technical Field]

The present invention relates to a method of differentiating pluripotent stem cells.

### [Background Art]

Hitherto, liver cell lines, primary cells and/or animal models have been used to demonstrate cellular and molecular mechanisms involved in the genesis of liver disease, to develop new treatments or toxicological risk assessment of substances. The results obtained with these different models can be criticized for several reasons. The most commonly used cell lines are either caused by cancer or immortalized and have genetic alterations leading to deregulation of major signaling pathways. The animal model is not entirely satisfactory, for reasons of cost of housing and because of ethical reasons. More importantly, the animal is not a good model for pharmaco-toxicological studies because it can predict effectively the toxicity of about 50 % of drugs. If primary hepatocytes are currently the reference models for the study of hepatic metabolism in vitro, it also presents a number of limitations. The number of donors is limited and sustainability/quality of obtained hepatocytes is low. In addition, the hepatocyte batch variability related to genetic polymorphism donor complicates standardized tests. Last points not least, hepatocytes do not proliferate in culture and eventually irreversibly lose their phenotype in culture. The development of an alternative source of mature and functional liver cells is essential.

Hepatocytes differentiated from pluripotent stem cells emerged as a promising source. Pluripotent stem cells have two main properties, they may differentiate into all cell types that make up the body (pluripotency) and are able in ad hoc conditions to proliferate indefinitely in culture (self-renewal). These cells thus represent a potentially inexhaustible source of mature and functional differentiated cells (see, for example, NPL 1).

### [Citation List]

### [Non Patent Literature]

NPL 1: R.E. Schwartz, et al. Pluripotent stem cell-derived hepatocyte-like cells. Biotechnology Advances 32 (2014):504-513.

### [Summary of Invention]

### [Technical Problem]

However, the aforementioned approach still does not give full satisfaction. HEP-LC (Hepatocytes Like Cells) still present a differentiation pattern of primitiveness (AFP, SOX17) illustrating that the maturation is not completed. Different hypotheses can be formulated to explain the immaturity of these cells: (i) the absence of interaction between these cells and the other component liver cells; (ii) the lack of hemodynamic stresses in the used system; (iii) the lack of organ to organ interaction; (iv) weak concentration of autocrine and paracrine factors in the cell culture. Sivertsson et al. (Stem cells and development, Vol. 22, N°4, 2013) discloses an improved method allowing to obtain a population consisting of HEP-LC by using 3-dimensional (3D) bioreaction system.

An object of the present invention is to provide a method of differentiating pluripotent stem cells.

### [Solution to Problem]

Accordingly, the present disclosure provides a method of differentiating pluripotent stem cells in liver and/or in liver-like cells including hepatocytes like cells (HEP-LC), endothelial like cells and biliary like cells, the method comprising steps of: seeding the pluripotent stem cells in a container provided with seeding medium; differentiating the pluripotent stem cells in the container; transferring cells from the container into a chamber of a bioreactor when said cells reach their progenitor stage where a premature hepatoblast is formed and where said cells are in a form of all the subtypes of premature liver like cells adhered together; and maturing the cells in the chamber, wherein the chamber includes a concave or a convex floor, fluid of medium flows in the chamber and oxygen is supplied into the chamber.

In another method, the bioreactor is loaded in a perfusion loop, in which the bioreactor is connected to a pump, and the loop is filled with the medium.

In yet another method, the bottom of the chamber includes an array of micro-chambers and micro-channels.

In yet another method, the pluripotent stem cells are human induced pluripotent stem cells.

As described herewith, the human induced pluripotent stem cells are differentiated into HEP-LC.

It is also described that the progenitor stage may be a stage where a definitive endoderm is formed, a stage where a specific hepatic pattern is formed.

As indicated above, the human induced pluripotent stem cells are transferred from the container into the chamber of the bioreactor in a form of all the subtypes of premature liver like cells adhered together.

Moreover, in the method of the present invention, the human induced pluripotent stem cells are matured in the chamber of the bioreactor in the form of all the subtypes of premature liver like cells adhered together.

### [Advantageous Effects of Invention]

According to the present disclosure, the pluripotent stem cells can be stably differentiated in a high cell density at a high growth ratio.

### [Brief Description of Drawings]

[Fig. 1]

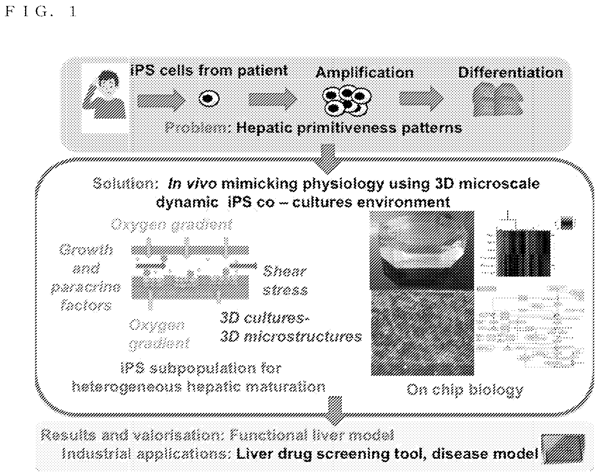

   FIG. 1 is a set of schematic views showing general concept.
[Fig. 2]

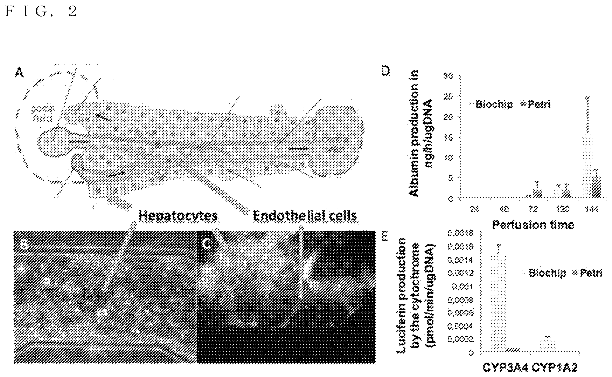

   FIG. 2 is a set of views showing general results.
[Fig. 3]

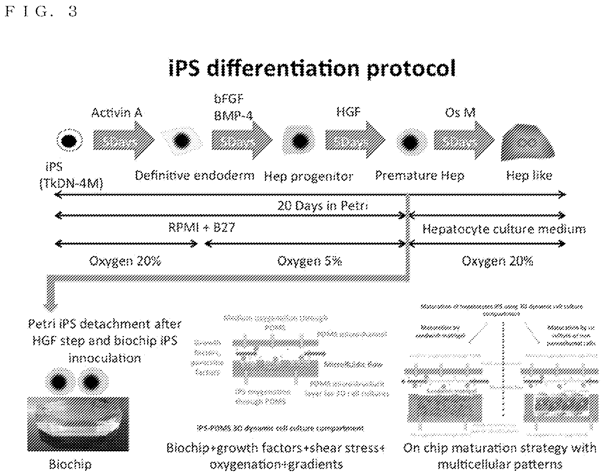

   FIG. 3 is a set of views showing iPS differentiation protocol.
[Fig. 4]

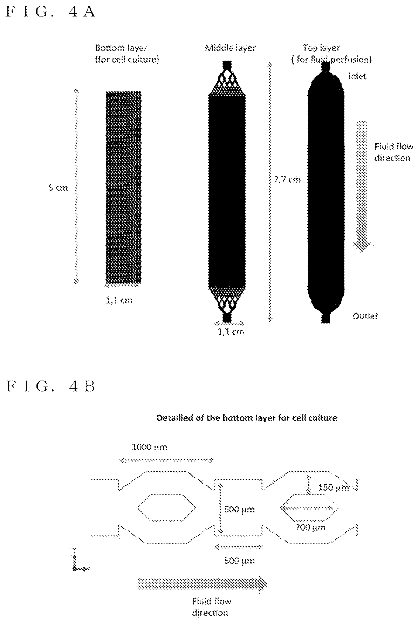

   FIG. 4 is a set of schematic views of design of bioreactor.
[Fig. 5]

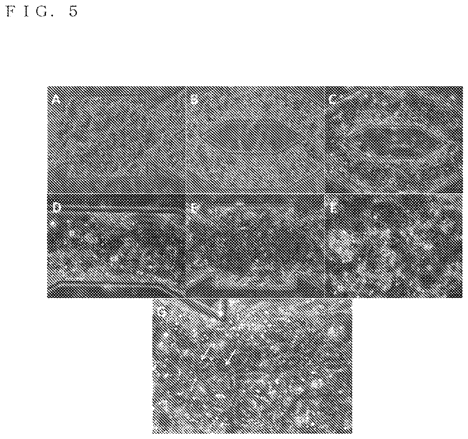

   FIG. 5 is a set of microphotographs showing tissue morphologies in bioreactor.
[Fig. 6]

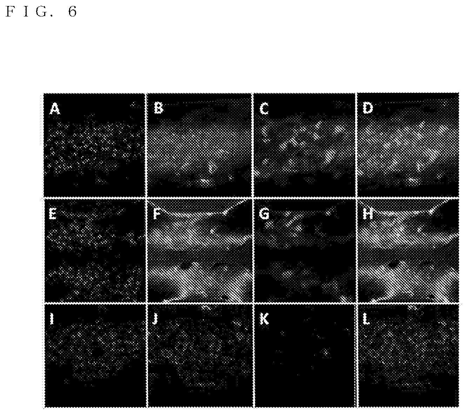

   FIG. 6 is a set of microphotographs showing bioreactor staining.
[Fig. 7]

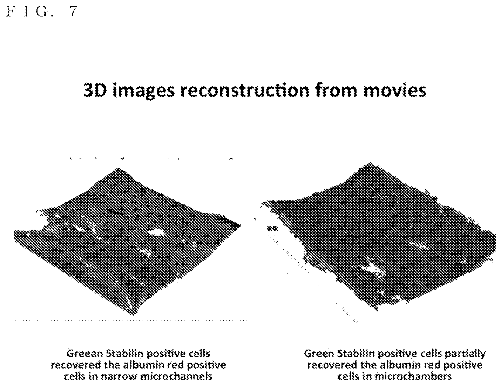

   FIG. 7 is a set of images of 3D reconstruction of cells in bioreactor.
[Fig. 8]

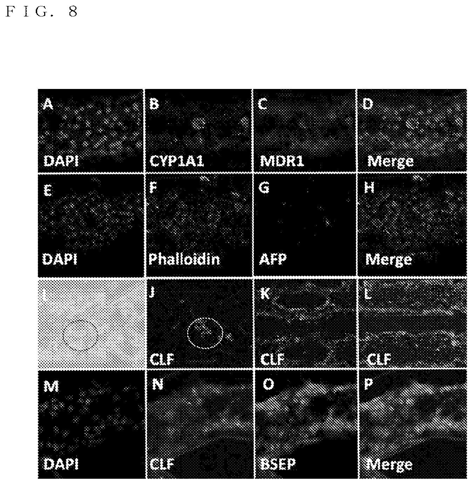

   FIG. 8 is another set of microphotographs showing bioreactor staining.
[Fig. 9]

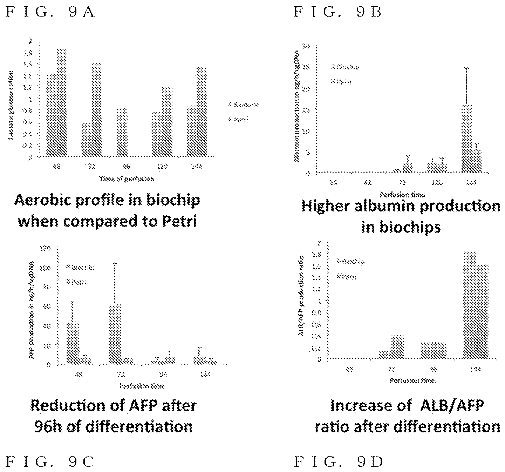

   FIG. 9 is a set of graphs showing results of functional assays.
[Fig. 10]

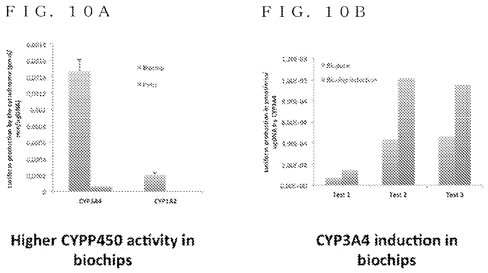

   FIG. 10 is a set of graphs showing results of CYP3A4 and CYP1A2 assays.

### [Description of Embodiments]

An embodiment will be described in detail with reference to the drawings.

FIG. 1 is a set of schematic views showing general concept. FIG. 2 is a set of views showing general results. FIG. 3 is a set of views showing iPS differentiation protocol.

The present embodiment proposes a new process of differentiation of pluripotent stem cells using biochemical and mechanical stimulation. The pluripotent stem cells, which can be differentiated according to the present embodiment, are preferably primed ones. In a category of the primed stem cells included are all of the pluripotent stem cells, the induced pluripotent stem (iPS) cells and embryonic stem (ES) cells, the mesenchymal stem cells, such as human iPS cells, human ES cells and human mES cells. Other animal sources are also suitable such as bovine, monkey, rodent stem cells. The present inventors used only human iPS cells for the sake of convenience in experiments in the present embodiments. Moreover, the human embryonic stem cells as used herein are obtained by conventional methods without destroying human embryo. Preferably, the method of the present invention uses induced pluripotent stem cells (iPSC). More preferably, iPSC are human iPSC.

One of its important points is to stop the conventional differentiation of the iPS in petri dishes, which are used here as containers, at an immature step of the final tissue target iPS cells. Therefore the iPS population is still heterogeneous with several population subtypes. Then the iPS cells are transferred into a bioreactor and the differentiation protocol is continued. The bioreactor environment provides additional stimuli to the iPS during the differentiation protocol when compared to the petri dish culture. The biochemical stimulation contributes to orientate the iPS differentiation to the selected tissue target. In parallel, the mechanical stimulation in the bioreactor contributes to orientate a part of cell population to a second lineage. According to experiments conducted by the present inventors, it was observed that high oxygenation, growth factors gradients and their local concentrations (paracrine or autocrine, endocrine) along the bioreactor enhanced selective differentiation and therefore the overall tissue maturation (as shown in FIG. 1). FIG. 1 illustrates a general concept strategy of the experiments based on liver maturation in microscale bioreactor (biochip) mimicking in vivo physiology such as control of (i) shear stress, (ii) oxygen gradient, (iii) iPS subpopulation, (iv) growth factors and (v) 3D cultures. In the case of liver differentiation using this protocol, the present inventors identified hepatic, endothelial and epithelial bile like sub lineage. The bioreactor and the proposed protocol act as an in vivo mimicking microenvironment (as shown in FIG. 2). FIG. 2 illustrates example of general results: (A) liver sinusoid, (B) mimicking human iPS hepatic co cultures, (C) red albumin and green stabilin positive cells, (D) albumin production, and (E) CYP450 activities in Biochip (bioreactor) and Petri (petri dish or container).

Pluripotent stem cells have two main properties, one of which is that they may differentiate into all cell types that make up the body (pluripotency), and the other of which is that they are able in ad hoc conditions to proliferate indefinitely in culture (self-renewal). These cells thus represent a potentially inexhaustible source of mature and functional differentiated cells. It is currently assumed that human pluripotent cells can be differentiated in liver cells, HEP-LC (Hepatocytes Like Cells). HEP-LC express major liver phenotypic markers, mimic hepatic metabolism, including those of xenobiotics. Despite these encouraging results, aforementioned problems are still remaining. Therefore the present inventors have integrated additional approaches to solve these problems. The protocol proposed in the present embodiment is based on a preliminary differentiation in a petri dish and then a maturation using a bioreactor and a sequence of stimulations (as shown in FIG. 3). FIG. 3 illustrates the protocol of differentiation based on the inoculation of immature iPS in a bioreactor (biochip) after the step 3 of petri dish differentiation.

It must be noted that the method of differentiating human induced pluripotent stem cells described herein concerns the differentiating in liver cells or HEP-LC but is also available to differentiating in cells for other organs, such as spleen, pancreas, heart and intestines. For the sake of convenience, objects of explanation in the present embodiment would be differentiating human induced pluripotent stem cells in liver cells or HEP-LC.

Next will be described a fabrication of bioreactor or biochip used in the present embodiment.

FIG. 4 is a set of schematic views of design of bioreactor.

The bioreactor (biochip) was fabricated by replica molding in polydimethylsiloxane (PDMS). The molds were built through double photolithography process using SU-8 photoresist. The bioreactor includes a cell culture chamber of 5 cm long and 1 cm wide. The height is 300 µm. In the bottom of the culture chamber, an array of micro-chambers and micro-channels are formed to enhance multilayer cell culture and microfluidic cell culture. As shown by the left end illustration in the middle section of FIG. 1 and by the second left illustration in the bottom section of FIG. 3, a cross sectional view of the floor surfaces of micro-chambers and micro-channels are rugged or in a shape of alternative connection of concave and convex parts in the direction of flow of growth factors. Therefore, iPS cells can be cultured in three dimensions (3D). Further, oxygen can be supplied through penetrating both ceiling and floor panels of PDMS so that population of iPS cells can grow rapidly. This basal unit of the design of this microarray is based on the previous work of Eric Leclerc, one of the present inventors (see NPL 2). Eric Leclerc proposed the design of the bioreactor (as shown in FIG. 4). The molds were built by LAAS facilities via the RTB program. PDMS bioreactor was fabricated in LIMMS/Pr Sakai laboratory. FIG. 4 illustrates the design of the bioreactor: (A) microstructure layer to create the bioreactor. Top layer is used to perfuse the culture medium. Bottom layer is used to cultivate the cells in microscale micro-chambers and micro-channels. Maximal depth is 300 µm. (B) a detail of the bottom layer including the periodic microstructures that are reproduced along the bioreactor information.

NPL 2: Audrey Legendre, et al. Investigation of the hepatotoxicity of flutamide. Toxicology in Vitro 28 (2014):1075-1087.

Next will be described a preliminary iPS cell differentiation protocol used in the present embodiment.

In the present embodiment, the protocol of iPS differentiation is based on the study of Duncan's group (see NPL 3). The iPS cells used for the experiment in the present embodiment were coming from the University of Tokyo. The protocol of Duncan's group was modified for a 24 well plates by Pr. Miyajima's group (work done by Pr. Kido). The present inventors used this protocol for 6 well plates. The 6 well petri dishes were coated with Matrigel (R) for one hour. After washed with culture medium, the iPS cells were seeded in the petri dishes. The seeding medium was mTeSR (R) complemented with anti-apoptotic agent. After 24 h in the seeding medium, the proliferation mTeSR (R) medium was used. When the iPS cells reached 90 % of confluence, the differentiation process started. For that purpose, the iPS cells were exposed in RPMI medium supplemented with B27 supplement and 100 ng/mL of Activin A for five days in order to form definitive endoderm (step 1: see the left end arrow in the top section of FIG. 3). Then the iPS cells were exposed for five days to bFGF and BMP4 at 10 and 20 ng/mL respectively to form the specific hepatic pattern in the RPMI+B27 medium (step 2: see the second left arrow in the top section of FIG. 3). At the end of the step 2, the cells were exposed to 20 ng/mL of HGF in RPMI+B27 medium to reach the hepatoblast progenitor (step 3: see the third left arrow in the top section of FIG. 3). In proliferation the culture medium was changed every day. In the steps 1, 2 and 3, the culture medium was changed after 24 h and 72 h of culture. The proliferation step and the step 1 were performed in 20 % O₂ and 5 % CO₂ incubator whereas the steps 2 and 3 were performed under 5 % O₂.

NPL 3: Karim Si-Tayeb, et al. Highly Efficient Generation of Human Hepatocyte-like Cells from Induced Pluripotent Stem Cells. Hepatology, 2010 January; 51(1):297-305.

Next will be described iPS cultures in the bioreactor used in the present embodiment.

The bioreactor was sterilized by autoclave before utilization. Its inner surfaces were coated with Matrigel (R) solution for one hour. After washed with culture medium, the iPS cells were loaded in the bioreactor (step 4: see all the illustrations in the bottom section of FIG. 3). To perform iPS hepatic maturation in the bioreactor, the iPS cells were detached from the petri dishes at the end of the step 3 of the differentiation. The inoculation density in the bioreactor was two times higher in the petri dishes in order to avoid low cell number in the bioreactor and subsequent de-differentiation. The cell adhesion was performed in an incubator at 20 % of oxygen and in the culture medium of the step 3 (with HGF), in which the anti-apoptotic substrate was added. Once the iPS cells were adhered, the bioreactor was loaded in a perfusion loop. The perfusion loop was a bubble trap, where the bioreactor and a pump were serially connected. The loop pipes were made of PTFE. The loop was filled with 3 mL of the step 4 medium. The step 4 medium was the supplemented Lonza medium with growth factors of the provider and additionally supplemented with 20 ng/mL of OSM. Flow rate was launched to perform dynamic culture at between 10 and 25 pL/min (preferably, 20 µL/min). One and a half mL of the culture medium was changed every day during the perfusion. The experiments were performed in a 5 % CO₂ and 20 % O₂ incubator for one week.

According to conventional methods of maturing premature hepatocytes like cells in petri dishes, it was usual to separate them into respective groups of subtypes, to mature each groups of cells separately into their matured stage and, finally, to put the groups of cells in matured stage together in order to get matured hepatocytes like cells. On the contrary, according to the present invention, it was possible to maturate plural subtypes of premature liver like cells, in a form of adhering all the subtypes of premature liver like cells together, into matured liver like cells in the bioreactor. The liver like cells include hepatocytes like cells, endothelial like cells, biliary like cells, and so forth. This could attribute to a fact that the density of the cells was locally high and the surface area per unit was large because the cells could be cultured in three dimensions in the micro-chambers and micro-channels of the bioreactor, and to another fact that the population of the cells grew rapidly because the medium was fluently provided therein as a form of flow and oxygen was enough supplied through PDMS panels. High cell density in a bioreactor chamber (several millions of cells in few microliters) contributes to locally concentrating the growth factors, including autocrine and paracrine factors. Heterogeneous microscale environment (micro-channels and micro-chambers) provides various local micro environments in which cells can adapt (such as endothelial cells elongation along walls or reorganization according to the local shear stress).

Next will be described the results of the experiments in the present embodiment. It will show that the protocol used in the present embodiment contributes to enhance the maturation of the hepatocytes and to create a more functional hepatic tissue when compared to conventional petri dish methods. First will be described the tissue heterogeneity.

FIG. 5 is a set of microphotographs showing tissue morphologies in bioreactor. FIG. 6 is a set of microphotographs showing bioreactor staining. FIG. 7 is a set of images of 3D reconstruction of cells in bioreactor. FIG. 8 is another set of microphotographs showing bioreactor staining.

When 96 h had passed from the start of perfusion, the present inventors could clearly observe cuboid hepatocyte like shape phenotypes in the center parts of the micro-chambers and micro-channels of the bioreactor. On the side of the micro-channels, elongated cells were observed. After 96 h of culture, hepatocyte like cells surrounded by fibroblastic morpho-type cells were largely observed overall in the micro-channels of the bioreactor. After 7 days of perfusion, the created tissue in the bioreactors formed a dense 3D like tissue (see FIG. 5). FIG. 5 illustrates tissue morphologies in the bioreactor and, therein, (A) and (B) illustrate those after adhesion, (C), (D), (E) and (F) illustrate those after 96 h of culture and (G) illustrates that after 144 h of culture.

Immunostaining of the tissues showed that the hepatocytes like cells were positive to albumin immunostaining (see FIG. 6). FIG. 6 illustrates bioreactor staining and, therein, (A), (E) and (I) illustrate those of cell nucleus, (B) and (F) illustrate those of stabilin positive cells, (C) and (G) illustrate those of albumin positive cells, (D) and (H) illustrate merger images of cell nucleus, stabilin and albumin, (J) illustrates that of phalloidin positive cells, (K) illustrates that of alpha-fetoprotein positive cells, and (L) illustrates a merger image of phalloidin and alpha-fetoprotein. The stabilin positive cells appeared to surround and embed the albumin positive cells in their area. In addition, the cells located on the top of the microstructures and of the tissue were stabilin positive but not albumin positive (see FIG. 6 and 7). FIG. 7 illustrates the 3D reconstruction from confocal image of the red albumin and green stabilin positive cells in the bioreactor.

The cholyl-lysyl-fluorescein (CLF) was secreted into bile canaliculi by a bile salt export pump (BSEP). The CLF staining generally shows numerous positive cells in the bioreactor cultures, but the dense bile like duct networks with the CLF accumulation was observed in the cellular aggregates which closed the wall of the microstructures (see FIG. 8). FIG. 8 illustrates staining in bioreactor showing some MDR1, CYP1A1, CLF and BSEP positive cells. Therein, DAPI shows the cell nucleus and AFP is weakly expressed. The CLF network like accumulation was weakly observed in petri dishes. In addition, the immunostaining of BSEP revealed that the bioreactor tissue was positive to this transporter, mainly on the microchannel sides. Superposition of BSEP and CLF accumulation confirmed that BSEP and CLF were largely co-localized.

Using a set of images from several bioreactors and experiments, the present inventors established a ratio of albumin positive cells versus the overall cell population based on the image processing. In bioreactors up to 60±8 % albumin positive cells were found whereas in petri dishes only 29±1 % albumin positive cells were found. In addition, based on FACS experiments the present inventors established that 36±6 % of cells were stabilin positive in the bioreactor cultures whereas larger dispersion was observed in petri dish cultures (23±23 %).

Next will be described the tissue functionality.

FIG. 9 is a set of graphs showing results of functional assays. FIG. 10 is a set of graphs showing results of CYP3A4 and CYP1A2 assays.

The levels of glucose consumption and lactate production were evaluated to get information on the respiration and glycolysis pathway status during the cultures (see FIG. 9). FIG. 9 illustrates functional basal assays in bioreactors and petri dishes and, therein, (A) illustrates lactate glucose ratio, (B) illustrates albumin production, (C) illustrates alpha-fetoprotein, and (D) illustrates albumin/alpha-fetoprotein ratio. After cell adhesion and 24 h of perfusion, in the bioreactor, the present inventors found an intense glucose consumption with a high lactate/glucose ratio, which illustrated an anaerobic respiration. After 48 h of perfusion, the lactate/glucose ratio remained below 1, closed to 0.8, this illustrated a healthy aerobic respiration in the overall tissue. Anaerobic profile was observed in petri dish cultures with ratio value ranging between 1.6 and 1.8 in the cases of the albumin productions were high.

In order to evaluate the cell functionality in regards of the level of maturation in the bioreactors and petri dishes, the present inventors have measured the production of albumin and alpha-fetoprotein. When the measured values were normalized by the number of hepatocytes in each culture, it was found that bioreactor production was higher in bioreactor than petri dishes. In parallel, the kinetics of the AFP/ALB ratio decreases with time for bioreactor, showing a continuous maturation of the hepatocytes in bioreactors (see FIG. 9).

To confirm the level of maturation of iPS hepatocytes like cells in bioreactors and the functional performance of the tissue, the present inventors performed CYP3A4 and CYP1A2 assays (see FIG. 10). FIG. 10(A) illustrates CYP3A4 and CYP1A2 activities measured by the production of luciferin from dedicated substrates, and FIG. 10(B) illustrates typical luciferin production by CYP3A4 following induction by rifampicin in bioreactor. For that purpose, the present inventors investigated the luciferin production from specific substrates in bioreactors and petri dishes. In all the petri dish cultures, the present inventors did not detect the luciferin production. Conversely luciferin was produced from the iPS cells cultivated in bioreactors. In addition, treatment of 25 µm of rifampicin contributed to induce the cells. In fact, treated cells doubled the production of luciferin when compared to untreated ones. petri dish cultures were not inducible.

Next will be described advantageous effects provided by the present embodiment.

One of the characteristic points of the present embodiment is a fact that iPS cells are able to be differentiated coupled with bioreactor and bioreactor technologies as microscale bio-artificial organs. Many groups are developing tissue engineered processes in order to provide a more appropriate environment for the hepatocytes maintenance and development. This environment has to reproduce as closely as possible the characteristics found in vivo. One of such in vitro systems can be made using recent developments in the field of micro technology to design micro scale in vivo mimicking devices. The cellular reorganization brought about by the micro topography of these systems plus the dynamic microfluidic culture conditions appears to be a key feature for reproducing 3D multi cellular in vivo situations. As an example of the methods potential, the present inventors presented preliminary results of liver iPS differentiation in their bioreactors. Multi cellular differentiation and early hepatocyte maturation were achieved in the bioreactors. In addition hepatic like span in bioreactor coupled with functional CYP450 activity was observed when compared to petri dishes. Although the bioreactor aspects are important, the present embodiment includes a pre-conditioning of the iPS using conventional petri dish pre-differentiation to get heterogeneous immature iPS cell subpopulation.

The present embodiment includes plural novel features, such as an iPS differentiation protocol using a multiplex stimulation including chemical stimulation via the endocrine growth factor, gradient of growth factor along the cell culture area, the mechanical stimulation via shear stress, an oxygen modulation via a permeable material used as a cell support, and a 3D cellular reorganization via a micro-structured support allowing high cell density over the surface and volume of cell culture area. All of these features can be included in the protocol using the microfluidic bioreactors.

Existing technologies and protocols are promoting iPS differentiation by adding specific growth factors in the culture medium in a well-defined sequence or by genetic modifications. The approach of the present embodiment provides additional types of stimulation to induce other cellular pathways involved in the stem cell differentiation.

The conventional protocols did not lead to mature liver hepatocytes. Similarly, when applied to other tissues, such as pancreas, the produced cells were weakly functional when compared to human mature tissues.

Next will be described utilization of the present embodiment.

The current status of primitiveness of hepatic iPS patterns is a one drawback to put liver iPS therapeutic solution into clinical trials. The limited availability of functional hepatocytes for drug testing is also reported as a major bottleneck bringing pharmaceutical companies to spend $1 billion/year on liver cells alone. The future ability to produce a supply of functional liver cells from human pluripotent stem cells can change this situation. At present the scale of the bioreactor used in the present embodiment is too small to think of large-scale production of functional cells for liver transplantation. The main application is at present the drug screening assays and thus the partial substitution to human primary cells in drugs assays.

The concept of the protocol using partially differentiated iPS cells before to cultivate them in a bioreactor mimicking in vivo physiology can be applied to numerous other targeted organs, such as pancreas, intestine or kidney. Other applications can be more generally related to the regenerative medicine and the personalized medicine. Indeed based on patient iPS cells harvesting, specific cell therapy or patients related diseases might be more pertinently investigated. Ultimately, if the protocol can be extended to large scale productions, it would be possible to produce enough cells for tissue and organs transplantation.

### [Industrial Applicability]

The present invention is applicable to a method of differentiating pluripotent stem cells.

## Claims

1. A method of differentiating pluripotent stem cells in liver and/or in liver-like cells including hepatocytes like cells (HEP-LC), endothelial like cells and biliary like cells, the method comprising steps of:
seeding the pluripotent stem cells in a petri dish provided with seeding medium;
starting to differentiate the pluripotent stem cells in the petri dish;
transferring cells from said petri dish into a chamber of a bioreactor when said cells reach their progenitor stage where a premature hepatoblast is formed and where said cells are in a form of all the subtypes of premature liver like cells adhered together.; and
maturing the cells in the chamber, where the cells are in the form of all the subtypes of premature liver like cells adhered together,
wherein the chamber includes a concave or a convex floor, fluid of medium flows in the chamber and oxygen is supplied into the chamber.

2. The method according to Claim 1, wherein the bioreactor is loaded in a perfusion loop, in which the bioreactor is connected to a pump, and the loop is filled with the medium.

3. The method according to Claim 2, wherein the bottom of the chamber includes an array of micro-chambers and micro-channels.

4. The method according to Claim 1, wherein the pluripotent stem cells are human induced pluripotent stem cells.

## Patentansprüche

1. Verfahren zum Differenzieren von pluripotenten Stammzellen in Leber- und/oder in leberähnlichen Zellen, einschließlich hepatozytenähnlichen Zellen (HEP-LC), endothelähnlichen Zellen und gallenähnlichen Zellen, wobei das Verfahren Schritte umfasst des:
Aussäens der pluripotenten Stammzellen in einer Petrischale, die mit Aussaatmedium versehen ist;
Beginnens, die pluripotenten Stammzellen in der Petrischale zu differenzieren;
Transferierens von Zellen aus der Petrischale in eine Kammer eines Bioreaktors, wenn die Zellen ihr Vorläuferstadium erreichen, in dem ein früher Hepatoblast gebildet wird, und in dem die Zellen in einer Form vorliegen, bei der alle die Subtypen früher leberähnlicher Zellen zusammenhaften; und
Reifen der Zellen in der Kammer, wobei die Zellen in der Form vorliegen, bei der alle die Subtypen früher leberähnlicher Zellen zusammenhaften,
wobei die Kammer einen konkaven oder einen konvexen Boden einschließt, Medienfluid in der Kammer fließt, und Sauerstoff in die Kammer zugeführt wird.

2. Verfahren nach Anspruch 1, wobei der Bioreaktor in eine Perfusionsschleife geladen wird, in der der Bioreaktor an eine Pumpe angeschlossen wird, und die Schleife mit dem Medium gefüllt wird.

3. Verfahren nach Anspruch 2, wobei der Boden der Kammer eine Anordnung von Mikrokammern und Mikrokanälen einschließt.

4. Verfahren nach Anspruch 1, wobei es sich bei den pluripotenten Stammzellen um human induzierte pluripotente Stammzellen handelt.

## Revendications

1. Méthode de différenciation de cellules souches pluripotentes en cellules hépatiques et/ou de type hépatique incluant des cellules de type hépatocytes (HEP-LC), des cellules de type endothélial et des cellules de type biliaire, le procédé comprenant les étapes suivantes :
l'ensemencement de cellules souches pluripotentes dans une boîte de Petri pourvue d'un milieu d'ensemencement ;
l'initiation de la différenciation des cellules souches pluripotentes dans la boîte de Pétri ;
le transfert des cellules depuis ladite boîte de Petri dans une chambre d'un bioréacteur quand lesdites cellules atteignent leur stade de progénitrice où un hépatoblaste prémature est formé et où lesdites cellules sont sous la forme de tous les sous-types de cellules de type hépatique prématures liées ensemble ; et
la maturation des cellules dans la chambre, où les cellules sont sous la forme de tous les sous-types de cellules de type hépatique prématures liées ensemble, dans laquelle la chambre inclut un plancher concave ou convexe, un fluide de milieu s'écoule dans la chambre et de l'oxygène est introduit dans la chambre.

2. Méthode selon la revendication 1, dans laquelle le bioréacteur est chargé dans une boucle de perfusion, dans laquelle le bioréacteur est raccordé à une pompe, et la boucle est remplie avec le milieu.

3. Méthode selon la revendication 2, dans laquelle le fond de la chambre inclut un réseau de micro-chambres et de micro-canaux.

4. Méthode selon la revendication 1, dans laquelle les cellules souches pluripotentes sont des cellules souches pluripotentes induites humaines.
